(19) Europäisches Patentamt
European Patent Office
Office européen des brevets

(11) Numéro de publication : **0 331 590 B1**

# (12) FASCICULE DE BREVET EUROPEEN

(45) Date de publication du fascicule du brevet : **14.04.93 Bulletin 93/15**

(51) Int. Cl.$^5$ : **C07C 45/28,** C07C 29/48

(21) Numéro de dépôt : **89420065.8**

(22) Date de dépôt : **21.02.89**

(54) **Procédé d'oxydation catalytique d'alcanes et d'hydrocarbures aralkyliques en mélanges d'alcools et de cétones.**

(30) Priorité : **03.03.88 FR 8802987**

(43) Date de publication de la demande : **06.09.89 Bulletin 89/36**

(45) Mention de la délivrance du brevet : **14.04.93 Bulletin 93/15**

(84) Etats contractants désignés : **AT BE CH DE ES FR GB GR IT LI LU NL SE**

(56) Documents cités :
**FR-A- 2 213 261**
**GB-A- 540 370**
**GB-A- 1 041 946**

(73) Titulaire : **RHONE-POULENC CHIMIE**
**25, quai Paul Doumer**
**F-92408 Courbevoie Cédex (FR)**

(72) Inventeur : **Costantini, Michel**
**10, Rue du Docteur Bonhomme**
**F-69003 Lyon (FR)**
Inventeur : **Lecomte, Jean-Pierre**
**24, Rue Boileau**
**F-69006 Lyon (FR)**

(74) Mandataire : **Vignally, Noel et al**
**Rhône-Poulenc Chimie Direction de la Propriété Industrielle Centre de Recherches des Carrières B.P. 62**
**F-69192 Saint-Fons Cédex (FR)**

**EP 0 331 590 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé d'oxydation catalytique d'alcanes en mélanges d'alcools et de cétones.

L'oxydation catalytique d'alcanes par les hydroperoxydes organiques est une réaction connue en elle-même et divers systèmes catalytiques ont déjà été proposés pour conduire cette réaction.

Ainsi, D. Mansuy et coll. dans Angew. Chem. Int. Ed. Engl. 19 (1980) n° 11, pages 909-910 ont décrit la décomposition de l'hydroperoxyde de cumyle dans le cyclohexane à 20°C, dans laquelle le catalyseur est le complexe Os(TPP) (CO) (Pyridine) où TPP désigne la tétraphénylporphyrine. Toutefois, le rendement en produits recherchés (cyclohexanol et cyclohexanone) est insignifiant et le catalyseur est très peu actif. Par ailleurs si sa structure est modifiée à 20°C, les travaux de la Demanderesse montrent qu' elle est détruite à une température plus élevée. Dans ces conditions, le développement à l'échelle industrielle d'une telle technique mettant en oeuvre un complexe au coût prohibitif est largement compromis.

Par ailleurs dans la demande de brevet français n° 2 559 154 est décrite notamment la dépéroxydation oxydante des hydroperoxydes de t-butyle et de cumyle dans le cyclohexane ou l'octane dans laquelle le catalyseur est un complexe du cobalt notamment porteur d'au moins un coordinat présentant le squelette de la bis(pyridyl-2′ imino) isoindoline. Il est également montré par l'exemple 27 de ladite demande que le composé $Co(Oct)_2$ de structure plus simple et qui ne porte pas un coordinat du type indiqué ci-avant ne présente qu'une très faible efficacité. Dans cette demande il est évoqué le possible remplacement du cobalt, métal central des complexes en cause, par l'un quelconque des métaux du groupe VIII, l'osmium étant l'une des possibilités envisagées. Toutefois les coordinats en cause sont relativement difficiles à préparer et l'efficacité de l'ensemble des complexes testés dans la réaction envisagée, dont le métal central est le cobalt, reste faible. Le développement à l'échelle industrielle d'une telle technique paraît donc également compromis.

Il était donc nécessaire de proposer un procédé d'oxydation catalytique d'alcanes par les hydroperoxydes organiques qui soit plus efficace, qui permette la mise en oeuvre de catalyseurs plus stables thermiquement, d'un accès plus aisé et susceptibles de pouvoir être, le cas échéant, recyclés.

La présente invention a donc pour objet un procédé d'oxydation d'alcanes en un mélange d'alcools et de cétones par un hydroperoxyde organique en présence d'un catalyseur à base d'osmium caractérisé en ce l'osmium est introduit sous forme d'osmium métallique, le cas échéant déposé sur un support, sous forme d'un composé minéral de l'osmium ou sous forme d'un complexe organique de l'osmium libérant dans le milieu réactionnel une forme minérale de l'osmium et en ce qu'on opère à une température comprise entre 50 et 180°C.

Par alcanes, substrats dans le cadre du présent procédé on entend des hydrocarbures saturés répondant à la formule (I) ci-après :

$$RH \quad (I)$$

dans laquelle R représente :

- un groupement alkyle linéaire ou ramifié qui comporte de 1 à 30 atomes de carbone,
- un groupement cycloalkyle qui comporte de 3 à 12 atomes de carbone dans le cycle, éventuellement substitué par un ou plusieurs groupements alkyles qui renferment au maximum 4 atomes de carbone,
- un groupement polycycloalkyle comportant de 2 à 5 cycles dont chaque cycle peut renfermer de 3 à 12 atomes de carbone, ou
- un groupement alkyl- ou cycloalkylaromatique qui comporte de 7 à 30 atomes de carbone.

Plus spécifiquement R représente :

- un groupement alkyle linéaire ou ramifié qui comporte de 1 à 12 atomes de carbone,
- un groupement cycloalkyle qui comporte de 5 à 12 atomes de carbone dans le cycle,
- un reste d'alkylbenzène dans lequel le groupe alkyle renferme au maximum 4 atomes de carbone, ou
- un reste de cycloalkylbenzène dans lequel le groupe cycloalkyle renferme de 5 à 8 atomes de carbone.

A titre d'exemples d'alcanes oxydables dans le cadre du présent procédé, on peut citer : le méthane, l'éthane, le propane, l'isobutane, l'isopentane, le butane, l'hexane, l'octane, le cyclopentane, le cumène, le toluène, la tétraline, la décaline, le cyclododécane et le pinane.

Dans le cadre du présent procédé on recourt également à un hydroperoxyde organique. Ce composé peut être représenté par la formule générale (II) ci-après :

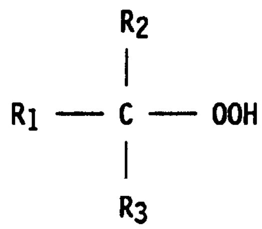

(II)

dans laquelle :

- $R_1$ à $R_3$, identiques ou différents, représentent :
  - * des atomes d'hydrogène,
  - * des groupements alkyles linéaires ou ramifiés qui comportent de 1 à 30 atomes de carbones,
  - * des groupements cycloalkyles qui comportent de 3 à 12 atomes de carbone,
  - * des groupements alkyl- ou cycloalkylaromatiques qui comportent de 7 à 30 atomes de carbone, ou
  - * des groupements aryles, éventuellement substitués par un ou deux groupement alkyles qui renferment au maximum 4 atomes de carbone, groupements aryles qui comportent de 6 à 20 atomes de carbone ;
- deux des groupements $R_1$ à $R_3$ peuvent en outre former ensemble un radiacal unique divalent alkylène renfermant de 4 à 10 atomes de carbone.

Plus spécifiquement $R_1$ à $R_3$, identiques ou différents représentent :

- des groupements alkyles qui comportent de 1 à 4 atomes de carbone
- des groupements phényles, ou
- pour deux d'entre-eux un radical unique divalent formant avec l'atome de carbone auquel il est relié un reste cyclohexyle ou cyclooctyle, ou
- pour l'un d'entre-eux, un atome d'hydrogène.

A titre d'exemples d'hydroperoxydes organiques convenant à la mise en oeuvre de la présente invention on peut citer l'hydroperoxyde de tertiobutyle, l'hydroperoxyde de cumyle, l'hydroperoxyde de cyclohexyle et l'hydroperoxyde d'éthylbenzène.

Comme indiqué en tête du présent mémoire, on obtient dans le cadre du présent procédé un mélange renfermant au moins l'alcool et au moins la cétone correspondant à l'alcane utilisé comme matière première ou à la partie alkylique du substrat aralkylique. Ainsi à partir du cyclohexane on obtiendra un mélange de cyclohexanol et de cyclohexanone qui sont des intermédiaires utiles de diverses fabrications (acide adipique, caprolactame) mélange communément désigné par OLONE. De même au départ d'éthylbenzène on obtiendra un mélange de phényl-1 éthanol et d'acétophénone utile pour la fabrication du styrène.

La mise en oeuvre du présent procédé requiert la présence d'osmium ou d'un composé de l'osmium.

N'importe quelle source d'osmium peut être utilisée dans le cadre de la présente invention. On peut, en effet, engager l'osmium sous forme métallique, le cas échéant, finement divisée ou déposée sur un support tel le charbon actif. Conviennent également à la mise en oeuvre dudit procédé, des composés de l'osmium dans lesquels l'osmium est au degré nul d'oxydation tel le triosmiumdodécacarbonyle. On peut également utiliser des composés minéraux de l'osmium dans lesquels l'osmium présente l'un quelconque des degrés d'oxydation 2 à 8. A titre d'exemples de tels composés on peut citer : OsO, $Os_2O_3$, $OsO_2$, $OsO_4$, $OsCl_3$, $K_2OsO_4$, $NaOsF_6$, $OsOCl_4$, $K_2OsO_4(OH)_2$, $OsCl_4$ et $OsOF_5$.

Il est également possible d'utiliser des composés organiques ou des complexes de l'osmium tels le tétracyclohexylosmium, le tétra(cyclohexyloxy) osmium ou le complexe Os(TPP)(CO)(Pyridine) et notamment, des complexes porteurs de coordinats denses en atome d'azote (par exemple tri- ou tétraazotés) tels les coordinats qui présentent le squelette de la tétraphénylporphyrine. La plupart des complexes en cause libèrent in situ dans les conditions de la réaction une forme minérale de l'osmium soit par dégradation des coordinats soit par décomplexation.

De préférence, on utilise l'une quelconque des formes d'osmium du groupe ci-après :

Os/C, $Os_3(CO)_{12}$, OsO, $Os_2O_3$, $OsO_2$, $OsO_4$ et $OsCl_3$.

Le tétroxyde d'osmium convient particulièrement bien à la mise en oeuvre de l'invention.

La quantité d'osmium à mettre en oeuvre n'est pas critique et elle peut varier dans de larges limites. Pour une bonne mise en oeuvre de l'invention elle sera d'au moins $10^{-6}$ mole d'osmium par mole d'hydroperoxyde et on n'observe aucun avantage à dépasser une quantité de $10^{-1}$ mole d'osmium par mole d'hydroperoxyde. Cette quantité est, de préférence, comprise entre $10^{-2}$ et $10^{-5}$ mole d'osmium par mole d'hydroperoxyde.

Le rapport molaire de l'hydroperoxyde à l'alcane peut lui aussi varier dans de larges limites ; un minimum de 0,001 % molaire est toutefois préconisé pour pouvoir observer un taux de transformation appréciable de

l'alcane, ce rapport pouvant atteindre 100 %. De préférence, ce rapport est compris entre 0,01 et 25 %.

Bien entendu la réaction peut être conduite avec un large excès d'alcane qui sert alors aussi de diluant. On peut également opérer en présence de diluants ou solvants non oxydables dans les conditions de la réaction, tels le benzène et le chlorobenzène.

Selon une variante avantageuse du présent procédé, la réaction est conduite en présence d'un diluant choisi parmi les alcools saturés, les diols et polyols saturés, l'eau et leurs mélanges.

Les alcools saturés qui conviennent à la mise en oeuvre du présent procédé répondent à la formule générale (III) ci-après :

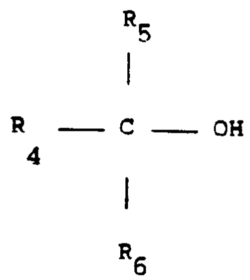

(III)

dans laquelle :

- $R_4$ à $R_6$, identiques ou différents, représentent :
  * des atomes d'hydrogène,
  * des groupements alkyles linéaires ou ramifiés qui comportent de 1 à 30 atomes de carbone,
  * des groupements cycloalkyles qui comportent de 3 à 12 atomes de carbone,
  * des groupements alkyl- ou cycloalkylaromatiques qui comportent de 7 à 30 atomes de carbone, ou
  * des groupements aryles, éventuellement substitués par un ou deux groupements alkyles qui renferment au maximum 4 atomes de carbone, groupements aryles qui comportent de 6 à 20 atomes de carbone ;
- deux des groupements $R_4$ à $R_6$ peuvent en outre former ensemble un radical unique divalent alkylène renfermant de 4 à 10 atomes de carbone,
- les trois groupements $R_4$ à $R_6$ peuvent en outre former ensemble un radical unique trivalent polycyclique renfermant de 6 à 20 atomes de carbone.

Les diols et le polyols saturés comportent un squelette répondant à la formule générale (III) ci-avant sur lequel est introduit au moins un groupe hydroxyle supplémentaire et, de préférence, au maximum 6 groupes hydroxyles supplémentaires.

A titre d'exemples de tels diluants on peut citer : le méthanol, l'éthanol, l'isopropanol, le tertiobutanol, l'hexanol-1, l'octanol-1, le dodécanol-1, le cyclohexanol, le diméthylphénylcarbinol, l'éthylèneglycol, le propanediol-1,3 et le diméthyl-2,4 dihydroxy-2,4 pentane.

Bien entendu l'alcool saturé utilisé comme diluant peut être de même nature ou de nature différente de l'alcool produit par la réaction ; il est également possible d'utiliser comme diluant un mélange renfermant un alcool endogène et un alcool exogène au milieu réactionnel.

De préférence, on utilise l'eau ou un alcool saturé répondant à la formule (III) ci-avant dans laquelle $R_4$ à $R_6$, identiques ou différents représentent des groupements alkyles linéaires qui comportent de 1 à 4 atomes du carbone ou, un atome d'hydrogène.

Le tertiobutanol convient plus particulièrement bien à la mise en oeuvre du procédé.

Comme indiqué ci-avant il est possible d'utiliser des mélanges de diluants et en particulier un mélange d'alcool saturé (ou de diol ou polyol saturé) et d'eau.

La quantité de diluant ou de mélange de tels diluants peut varier dans de larges limites ; une influence sensible est constatée dès lors que cette quantité représente de l'ordre de 2 % en poids de l'alcane à oxyder et aucun effet positif n'est observé lorsque cette quantité dépasse 200 % en poids de l'alcane. De bons résultats sont obtenus pour une quantité de diluant (s) comprise entre 2 et 100 % en poids de l'alcane.

Lorsqu'on utilise un mélange d'alcool et d'eau, la quantité d'eau n'est pas critique ; elle peut varier dans de larges limites.

Selon la teneur précise en eau du milieu réactionnel, la nature précise de l'alcool et/ou de l'alcane on peut observer la présence d'une seule phase ou de deux phases : une phase organique et une phase aqueuse. La présence d'un tel système biphasique notamment en fin de réaction est un autre avantage lié au procédé de la présente invention dans la mesure où l'on peut séparer les produits d'oxydation et l'alcane qui n'a pas réagi du système catalytique dont la majeure partie se trouve en fin de réaction dans la phase aqueuse, par décantation ou extraction. La phase aqueuse résiduelle peut être aisément utilisée, le cas échéant après traitement, pour catalyser une nouvelle réaction d'oxydation.

La Demanderesse a constaté qu'il est également avantageux d'opérer en présence d'un mélange tampon de telle sorte que le pH de la phase aqueuse soit maintenu entre 2 et 14. Pour ce faire, elle préconise notamment l'addition au milieu réactionnel d'un ou plusieurs composés choisis parmi : les hydroxydes des métaux alcalins, les oxyacides minéraux ou organiques et leurs sels des métaux alcalins ou des métaux alcalino terreux et en particulier, l'acide acétique et ses sels, l'acide phosphorique et ses sels, et, l'acide borique et ses sels.

La température de réaction dépend de la nature précise de l'alcane à oxyder et de celle de l'hydroperoxyde organique. Elle est généralement comprise entre 50 et 180°C et, de préference entre 70 et 150°C.

On opère à pression atmosphérique ou le cas échéant sous pression supérieure à la pression atmosphérique de manière à maintenir les constituants du mélange réactionnel en phase liquide.

La durée de réaction (ou le temps de séjour) généralement comprise entre quelques minutes et plusieurs heures peut être ajustée, compte-tenu des objectifs de production, de la quantité de catalyseur et des autres paramètres réactionnels.

En fin de réaction les produits peuvent être récupérés par tout moyen approprié, par exemple par distillation.

Les exemples ci-après illustrent l'invention.

Les conventions suivantes y sont utilisées :

- TT désigne : le taux de transformation de l'hydroperoxyde organique engagé à la réaction.
- RT désigne : le rendement en un produit (ou mélange de produits) par rapport à l'hydroperoxyde transformé.
- RT (OLONE) : désigne le rendement en mélange du cyclohexanol et de cyclohexanone.
- RT(OL) : désigne le rendement en cyclohexanol.
- RT(8-OL) : désigne le rendement en cyclooctanol.
- RT(8-ONE) : désigne le rendement en cyclooctanone.
- RT(8-OLONE) : désigne le rendement en mélange de cyclooctanol et de cyclooctanone.
- RT(DMPC) : désigne le rendement en diméthylphénylcarbinol.
- RT(A+D) : désigne le rendement en mélange de l'acétophénone et du diméthylphénylcarbinol.
- HPOC : désigne l'hydroperoxyde de cumyle
- HPOCH : désigne l'hydroperoxyde de cyclohexyle
- T : désigne la température

EXEMPLE 1 :

Dans un tube de verre, de type Carius, de 20 ml on charge à température ambiante :

- 28 mg (0,1 mmol) d'$OsO_4$ en solution dans le cyclohexane.
- 5 ml (46 mmol) de cyclohexane désoxygéné à l'argon et,
- 100 mg (0,9 mmol) d'hydroperoxyde de cyclohexyle.

On ajoute un barreau magnétique et purge le tube par un courant d'argon. On refroidit par carboglace, et on scelle le tube. L'ensemble est alors porté à 100°C pendant 22 Heures. La masse réactionnelle est alors analysée par iodométrie et par chromatographie en phase gazeuse.

99 % de l'hydroperoxyde de cyclohexyle a été converti :

RT (OL) = 89 %
RT (ONE) = 46 %
RT (OLONE) = 135 %

EXEMPLE 2 :

Dans une fiole de verre de 1,9 ml, bouchée par un opercule revêtu de Téflon[R], on charge :

- 0,48 mg d'osmium déposé sur charbon à 5 % (24 microgrammes d'osmium, soit $1{,}26.10^{-4}$ mmol d'osmium)
- 0,82 g (7,32 mmol) de cyclooctane
- 10,5 mg d'orthodichlorobenzène (étalon pour la CPG)
- 11,5 mg (0,75 mmol) d'hydroperoxyde de cumyle (HPOC) à 99 %.

La fiole est plongée dans un bain d'huile thermostaté à 100°C pendant 17 heures, le milieu réactionnel étant agité par un barreau magnétique.

Le taux de transformation de l'HPOC est de 100 % et les résultats obtenus sont les suivants :

RT(8-OL) = 18 %

RT(8-OLONE) = 38 %
RT(DMPC) = 80 %
RT(A+D) = 84,5 %

EXEMPLE 3 :

Dans un réacteur en verre de 200 ml de capacité, muni d'un réfrigérant descendant, d'une arrivée et d'une sortie de gaz, d'un bouchon septum, agité par un barreau magnétique et purgé à l'argon on introduit :

- 21 g (187 mmol) de cyclooctane
- 8 g (108 mmol) de tertiobutanol
- 2,5 ml du tampon 1 M $H_3BO_3$/NaOH (pH = 12,7)
- 6,7 $10^{-4}$ mmol d'osmium sous forme de triosmiumdodécarbonyle, en solution dans le chlorobenzène (0,20 mg de solution).

Le milieu réactionnel est porté à 80°C puis, on injecte à travers le bouchon septum 0,79 g (5,15 mmol) d'hydroperoxyde de cumyle (HPOC) en 20 secondes.

Le taux de transformation de l'HPOC est suivi par iodométrie ; en fin d'essai la masse réactionnelle homogénéisée par ajout de tertiobutanol est analysée par chromatographie en phase gazeuse.

Après 3,1 heures de réaction en température les résultats sont les suivants :

TT (HPOC) = 97 %
RT (8-OL) = 56,6 %
RT (8-ONE) = 8,1 %
RT (A + D) = 97,6 %

EXEMPLE 4 à 8 :

Dans un tube de verre et selon un mode opératoire analogues à ceux décrits pour l'exemple 1 on réalise une série d'essais sur une charge renfermant :

- 5,1 g (60,7 mmol) de cyclohexane
- 0,40 g (3,3 mmol) d'hydroperoxyde de cyclohexyle à 96 %
- du tertiobutanol
- du tétroxyde d'osmium en solution dans le cyclohexane

La durée de chaque essai est de 24 h.

Les conditions particulières ainsi que les résultats obtenus figurent dans le tableau I ci-après :

TABLEAU I :

| Réf | OsO4/HPOCH mol/mol | t-BuOH* (%) | T °C | TT (%) | RT(OL) (%) | RT(OLONE) (%) |
|---|---|---|---|---|---|---|
| 4 | 1,9.10-4 | 21 | 130 | 92,3 | 128,2 | 153,8 |
| 5 | 1,3.10-4 | 20 | 150 | 100 | 108,1 | 133,8 |
| 6 | 1,5.10-4 | 102 | 150 | 100 | 111,9 | 129,0 |
| 7 | 47.10-4 | 22 | 120 | 100 | 132,7 | 151,6 |
| 8 | 47.10-4 | 86 | 120 | 100 | 132,8 | 146,5 |

* t-BuOH : Rapport pondéral du t-BuOH au cyclohexane.

EXEMPLES 9 et 10 :

Dans une fiole de verre de 1,9 ml, bouchée par un opercule revêtu de Téflon[R], on charge :

- 1,10 g (13,1 mmol) de cyclohexane
- 0,0144 g (0,124 mmol) d'hydroperoxyde de cyclohexyle à 96 %
- $1{,}01.10^{-4}$ mmol de tétroxyde d'osmium en solution dans le cyclohexane
- 0,082 g de dichlorobenzène (étalon pour la CLHP)
- 0,047 g (0,632 mmol) de tertiobutanol pour l'exemple 9 seulement.

La fiole est plongée dans un bain d'huile thermostaté à 120°C pendant 20 heures. Les résultats et les conditions particulières figurent au tableau II ci-après. Le taux de transformation de l'HPOCH est de 100 % dans les deux cas.

TABLEAU II :

| Réf | $OsO_4$/HPOCH mol/mol | t-BuOH | RT(OL) (%) | RT(OLONE) (%) |
|---|---|---|---|---|
| 09 | $8{,}14.10^{-4}$ | NON | 134 | 159 |
| 10 | $8{,}14.10^{-4}$ | OUI | 161 | 173 |

EXEMPLES 11 à 13 :

Dans le réacteur et selon le mode opératoire analogues à ceux décrits pour l'exemple 3, on réalise une série d'essais, étant précisé que dans leur cadre il n'est plus procédé à une homogénéisation de la masse réactionnelle en fin d'essai, sur une charge renfermant :

- 30 g (268 mmol) de cyclooctane
- 7,7 g (104 mmol) de t-butanol
- 0,12 g (6,7 mmol) d'eau permutée
- du tétroxyde d'osmium en solution dans le cyclooctane dont la quantité est indiquée dans le tableau (III) ci-après :

La température étant portée à 80°C on injecte en 20 secondes :

- 1,6 g (10,4 mmol) d'hydroperoxyde de cumyle à 99 % (HPOC).

Les résultats obtenus au bout de 5 heures de réaction figurent également dans le tableau II ci-après, le taux de transformation de l'HPOC étant de 100 % dans tous les cas.

TABLEAU III:

| Réf | OsO4/HPOC mol/mol | RT(8-OL) (%) | RT(8-OLONE) (%) | RT(DMPC) (%) | RT(A+D) (%) |
|---|---|---|---|---|---|
| 11 | 8,8.10-2 | 46 | 77 | 94 | 100 |
| 12 | 7,9.10-3 | 54 | 78 | 93 | 100 |
| 13 | 8,4.10-5 | 75 | 81,8 | 92 | 100 |

EXEMPLES 14 à 18 :

Dans le réacteur et selon le mode opératoire analogues à ceux décrits pour l'exemple 3, on réalise une série d'essais, étant précisé que dans leur cadre il n'est plus procédé à une homogénéisation de la masse réactionnelle en fin d'essai, sur une charge renfermant :

- 30 g (268 mmol) de cyclooctane
- 8,4.$10^{-4}$ mmol de tétroxyde d'osmium en solution dans le cyclohexane et le cas échant :
- du tertiobutanol et
- de l'eau permutée.

La température étant portée à 80°C, on injecte en 20 secondes 1,6 g (10,4 mmol) d'hydroperoxyde de cumyle. (Le milieu réactionnel est homogène.

Les conditions particulières ainsi que les résultats obtenus figurent dans le tableau IV ci-après, le taux de transformation de l'HPOC étant de 100 % dans tous les cas.

TABLEAU IV :

| Réf | t-BuOH(*) (% pds) | H2O(*) (%pds) | Durée (h) | RT (8-OL) (%) | RT (8-OLONE) (%) | RT (DMPC) (%) | RT (A+D) (%) |
|---|---|---|---|---|---|---|---|
| 14 | 0 | 0 | < 1,5 | 26,3 | 46,8 | 88,2 | 92,2 |
| 15 | 2,7 | 0 | < 1 | 40,4 | 55,1 | 89,3 | 94,2 |
| 16 | 2,8 | 0,043 | 1,5 | 45,4 | 59,1 | 91,2 | 95,9 |
| 17 | 25,4 | 0 | 4 | 72,0 | 79,7 | 92,8 | 100 |
| 13 | 26 | 0,4 | 5,0 | 75,0 | 81,8 | 91,9 | 100 |
| 18 | 100 | 0 | 5,0 | 76,8 | 82,7 | 85,7 | 100 |

* Rapport pondéral au cyclooctane

EXEMPLE 19 :

On reproduit l'exemple 17 ci-avant en remplaçant le t-butanol par une quantité identique d'isopropanol. Les résultats obtenus sont les suivants :

TT (HPOC) = 100 %
RT (8-OL) = 55,8 %
RT (8-OLONE) = 58,8 %
RT (DMPC) = 94,2 %
RT (A+D) = 100 %.

EXEMPLE 20 :

On reproduit l'exemple 17 ci-avant en remplaçant le t-butanol par une quantité identique de méthanol. Les résultats obtenus sont les suivants :

TT (HPOC) = 100 %
RT (8-OL) = 60 %
RT (8-OLONE) = 66 %
RT (DMPC) = 95 %
RT (A+D) = 99 %.

EXEMPLE 21 :

Selon le mode opératoire décrit dans les exemples 11 à 13 on charge :

- 30 g (250 mmol) de cumène
- $7{,}0.10^{-4}$ mmol d'osmium sous forme de tétroxyde d'osmium en solution dans le cyclohexane.

La température étant portée à 100°C, on injecte en 20 secondes 1,71 g (11,2 mmol) d'hydroperoxyde de cumyle (HPOC) à 99 %. Au bout de 2h 30 mn la température est portée à 130°C. Le mélange est analysé après 6 h 20 mn de réaction. Les résultats sont les suivants :

TT(HPOC) = 98 %
RT(DMPC) = 115 %

RT (A+D) = 165 %

EXEMPLE 22 :

Dans une fiole en verre de 1,9 ml bouchée par un opercule revêtu de Téflon[R], on charge :

- 0,659 g (7,65 mmol) de n-hexane
- 0,0072 g (0,047 mmol) d'hydroperoxyde de cumyle à 99 %
- $7,72.10^{-5}$ mmol de tétroxyde d'osmium en solution dans le cyclohexane
- 0,0074 g d'orthodichlorobenzène (étalon pour la CPG)
- 0,234 g (3,15 mmol) de tertiobutanol

La fiole est plongée dans un bain d'huile thermostaté à 120°C pendant 18 heures.
Les résultats obtenus sont les suivants :

RT (hexanal+ hexanone-2) = 1,5 %
RT (hexanone-3) = 1,1 %
RT (hexanol-1) = 1,8 %
RT (hexanol-2) = 29,5 %
RT (hexanol-3) = 25,3 %
RT (hexanol + hexanone) = 59,2 %
RT (DMPC) = 71 %
RT (A+D) = 100 %

EXEMPLES 23 à 26 :

Dans une fiole de verre de 1,9 ml, bouchée par un opercule revêtu de Téflon[R] on charge :

- 0,755 g (8,95 mmol) de cyclohexane
- 0,025 g (0,163 mmol) d'hydroperoxyde de cumyle à 99 %.
- $3,8.10^{-5}$ mmol de tétroxyde d'osmium en solution dans le cyclohexane.
- du tertiobutanol
  (Le milieu est homogène).

La fiole est plongée dans un bain d'huile thermostaté à 100°C pendant 40 heures.
Le taux de transformation de l'HPOC est de 100 %.
Les conditions particulières ainsi que les résultats obtenus figurent dans le Tableau (V) ci-après :

TABLEAU V :

| Réf | t-BuOH(*) (% pds) | RT(OL) (%) | RT (OLONE) (%) | RT(DMPC) (%) | RT(A+D) (%) |
|---|---|---|---|---|---|
| 23 | 0 | 70.2 | 84.5 | 91.4 | 97.1 |
| 24 | 3,3 | 84.5 | 94.1 | 95.0 | 100 |
| 25 | 23 | 92.5 | 97.9 | 90.8 | 100 |
| 26 | 63 | 88.3 | 93.0 | 81.5 | 100 |

* Rapport pondéral au cyclohexane.

EXEMPLE 27 :

Dans la fiole et selon le mode opératoire décrit pour les exemples 23 à 26 on réalise un essai sur une charge

constituée par :

- 0,800 g (7,15 mmol) de cyclooctane
- 0,034 g (0,226 mmol) d'hydroperoxyde de cumyle à 99 %
- $1,9.10^{-5}$ mmol de tétroxyde d'osmium en solution dans le cyclohexane.
- 0,022 g (0,166 mmol) de diméthyl-2,4 dihydroxy-2,4 pentane.

Le taux de transformation de l'HPOC est de 100 %.

- RT (8-OL) = 47,7 %
- RT (8-OLONE) = 59,5 %
- RT (DMPC) = 86,1 %
- RT (A+D) = 92,2 %

EXEMPLE 28 :

On reproduit l'exemple 27 ci-avant à ceci près que l'on a chargé une quantité molaire équivalente de trichlorure d'osmium en solution dans le tertiobutanol au lieu du tétroxyde, et 640 mg (7,6 mmol) de tertiobutanol au lieu du diol.

Le taux de transformation de l'HPOC est de 100 %.

- RT (8-OL) = 68,6 %
- RT (8-OLONE) = 74,4 %
- RT (A+D) = 92,0 %

EXEMPLES 29 à 33 :

Dans le réacteur et selon le mode opératoire décrits pour l'exemple 3 on réalise une série d'essais en injectant pour chacun d'entre eux en 20 secondes 0,79 g (5,15 mmol) d'hydroperoxyde de cumyle à 99 % sur une charge renfermant :

- 21 g (187 mmol) de cyclooctane
- du tétroxyde d'osmium dont la quantité précise figure au tableau VI ci-après
- 2,5 ml d'une phase aqueuse et, le cas échant,
- 8,0 g (108 mmol) de t-butanol, et maintenue à 80°C.

La phase aqueuse (PA1) est constituée d'eau permutée

La phase aqueuse (PA2) est constituée par une solution molaire du tampon $H_3BO_3$/NaOH dans de l'eau permutée (pH = 12,7).

La phase aqueuse (PA3) est constituée par une solution molaire du tampon CHF3COOH/NaOH dans de l'eau permutée (pH = 5,3).

Les conditions particulières ainsi que les résultats obtenus sont rassemblés au tableau VI ci-après :

TABLEAU VI :

| Réf | OsO4/HPOC mol/mol | tBuOH | (*) | durée (h) | TT(HPOC) (%) | RT(8-OL) (%) | RT(8-OLONE) (%) |
|---|---|---|---|---|---|---|---|
| 29 | $1,12.10^{-4}$ | oui | PA1 | 0,5 | 98 | 64,7 | 66,7 |
| 30 | $1,10.10^{-4}$ | oui | PA1 | 7,0 | 33 | nd | nd |
| 31 | $1,10.10^{-4}$ | oui | PA2 | 3,0 | 97 | 59,5 | 67,7 |
| 32 | $1,10.10^{-4}$ | non | PA2 | 5,2 | 99,7 | 20,4 | 28,1 |
| 33 | $1,60.10^{-3}$ | oui | PA3 | 8,0 | 98,3 | 82,7 | 86,1 |

(*) : nature de la phase aqueuse utilisée

nd : non déterminé

EXEMPLE 34 :

Dans un réacteur en verre, équipé d'un réfrigérant, d'un barreau magnétique et d'un déversoir limitant la masse réactionnelle à 72 ml, on introduit :

- hydropéroxyde de cyclohexyle 96 % : 0,351 g ( 2,9 mmol)
- cyclohexanone : 0,482 g ( 4,91 mmol)
- cyclohexanol : 3,194 g (31,9 mmol)
- cyclohexane : 54 g
- $OsO_4$ en solution cyclohexanique : 0,0112 g ( 0,044 mmol)

Le milieu réactionnel est agité et chauffé à reflux (81°C).

Après 10 minutes, la masse est de couleur noire.

On injecte alors simultanément, par des tubulures en téflon :

- $OsO_4$ en solution cyclohexanique à 1,00 mmol/l : 6,91 g/h (8,9 micromol/h)
- Hydropéroxyde de cyclohexyle en solution cyclohexanique à 6 % en poids : 414 mmol/l : 117 g/h (60,7 mmol/h)
- Le reflux est maintenu par chauffage.

Après deux heures de fonctionnement, le régime permanent est atteint, le flux sortant par le déversoir est alors collecté et analysé.

Rapport molaire HPOCH introduit/Osmium introduit : 6974

Temps de séjour : 26,5 minutes

TT (HPOCH) : 99,2 % ({HPOCH} résiduaire/{cyclohexane} = $3,5.10^{-4}$)

activité de l'osmium : 4,3 cycles catalytiques/seconde

RT (6-ONE) : 30 %

RT (6-OL) : 105,5 %

RT (6-OLONE) : 135,5 %

## Revendications

1. Procédé d'oxydation catalytique de composés de formule générale :

RH (I)

dans laquelle R représente :

- un groupement alkyle linéaire ou ramifié qui comporte de 1 à 30 atomes de carbone,
- un groupement cycloalkyle qui comporte de 3 à 12 atomes de carbone dans le cycle, éventuellement substitué par un ou plusieurs groupements alkyles qui renferment au maximum 4 atomes de carbone,
- un groupement polycycloalkyle comportant de 2 à 5 cycles dont chaque cycle peut renfermer de 3 à 12 atomes de carbone, ou
- un groupement alkyl- ou cycloalkylaromatique qui comporte de 7 à 30 atomes de carbone,

en un mélange d'alcools et de cétones par un hydroperoxyde organique en présence d'un catalyseur à base d'osmium caractérisé en ce que l'osmium est introduit sous forme d'osmium métallique, le cas échéant déposé sur un support, sous forme d'un composé minéral de l'osmium ou sous forme d'un complexe organique de l'osmium libérant dans le milieu réactionnel une forme minérale de l'osmium, et en ce qu'on opère à une température comprise entre 50 et 180°C.

2. Procédé selon la revendication 1, caractérisé en ce que l'hydroperoxyde organique a pour formule générale :

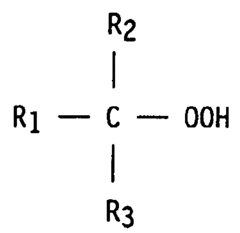

(II)

dans laquelle :

- $R_1$ à $R_3$, identiques ou différents, représentent :
  * des atomes d'hydrogène,
  * des groupements alkyles linéaires ou ramifiés qui comportent de 1 à 30 atomes de carbone,
  * des groupements cycloalkyles qui comportent de 3 à 12 atomes de carbone,
  * des groupements alkyl- ou cycloalkylaromatiques qui comportent de 7 à 30 atomes de carbone, ou
  * des groupements aryles, éventuellement substitués par un ou deux groupements alkyles qui renferment au maximum 4 atomes de carbone, groupements aryles qui comportent de 6 à 20 atomes de carbone ;
- deux des groupements $R_1$ à $R_3$ peuvent en outre former ensemble un radical unique divalent alkylène renfermant de 4 à 10 atomes de carbone.

3. Procédé selon l'une quelconque des revendications précédentes, caractérisé en ce que les composés de formule (I) donnée dans la revendication 1, sont choisis parmi ceux pour lesquels R représente :
   - un groupement alkyle linéaire ou ramifié qui comporte de 1 à 12 atomes de carbone,
   - un groupement cycloalkyle qui comporte de 5 à 12 atomes de carbone dans le cycle,
   - un reste d'alkylbenzène dans lequel le groupe alkyle renferme au maximum 4 atomes de carbone, ou
   - un reste de cycloalkylbenzène dans lequel le groupe cycloalkyle renferme de 5 à 8 atomes de carbone.

4. Procédé selon l'une quelconque des revendications précédentes, caractérisé en ce que l'hydroperoxyde organique répond à la formule (II) donnée dans la revendication 2 dans laquelle $R_1$ à $R_3$, identiques ou différents représentent :
   - des groupements alkyles qui comportent de 1 à 4 atomes de carbone
   - des groupements phényles, ou
   - pour deux d'entre-eux un radical unique divalent formant avec l'atome de carbone auquel il est relié un reste cyclohexyle ou cyclooctyle, ou
   - pour l'un d'entre-eux, un atome d'hydrogène.

5. Procédé selon l'une quelconque des revendications précédentes caractérisé en ce qu'on opère en présence d'un diluant choisi parmi les alcools saturés, les diols et polyols saturés, l'eau et leurs mélanges.

6. Procédé selon la revendications 5, caractérisé en ce qu'on opère en présence d'un alcool de formule :

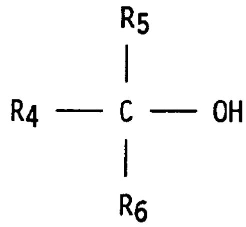

(III)

dans laquelle :

- $R_4$ à $R_6$, identiques ou différents représentent :
  - ∗ des atomes d'hydrogène,
  - ∗ des groupements alkyles linéaires ou ramifiés qui comportent de 1 à 30 atomes de carbone,
  - ∗ des groupements cycloalkyles qui comportent de 3 à 12 atomes de carbone,
  - ∗ des groupements alkyl- ou cycloalkylaromatiques qui comportent de 7 à 30 atomes de carbone, ou
  - ∗ des groupements aryles, éventuellement substitués par un ou deux groupements alkyles qui renferment au maximum 4 atomes de carbone, groupements aryles qui comportent de 6 à 20 atomes de carbone ;
- deux des groupements $R_4$ à $R_6$ peuvent en outre former ensemble un radical unique divalent alkylène renfermant de 4 à 10 atomes de carbone,
- les trois groupements $R_4$ à $R_6$ peuvent en outre former ensemble un radical unique trivalent polycyclique renfermant de 6 à 20 atomes de carbone.

7. Procédé selon la revendication 6, caractérisé en ce que l'alcool répond à la formule (III) dans laquelle $R_4$ à $R_6$, identiques ou différents représentent des groupements alkyles linéaires qui comportent de 1 à 4 atomes de carbone ou, un atome d'hydrogène.

8. Procédé selon l'une quelconque des revendications 5 à 7 caractérisé en ce que l'alcool représente de 2 à 200 % et, de préférence de 2 à 100 % en poids du composé de formule (I) donnée dans la revendication 1.

9. Procédé selon l'une quelconque des revendications 5 à 8 caractérisé en ce qu'on opère en présence d'un mélange d'alcool et d'eau.

10. Procédé selon l'une quelconque des revendications 5 à 9, caractérisé en ce que l'alcool utilisé est le tertiobutanol.

11. Procédé selon l'une quelconque des revendications précédentes caractérisé en ce que la quantité d'osmium est comprise entre $10^{-2}$ et $10^{-5}$ mole par mole d'hydroperoxyde.

12. Procédé selon l'une quelconque des revendications précédentes caractérisé en ce que l'hydroperoxyde organique représente de 0,001 à 100 % (molaire) du composé de formule (I) donnée dans la revendication 1, et de préférence, de 0,01 à 25 % (molaire).

13. Procédé selon l'une quelconque des revendications précédentes caractérisé en ce que la température de réaction est comprise entre 70 et 150°C.

14. Procédé selon l'une quelconque des revendications précédentes caractérisé en ce que l'osmium est introduit sous forme de son tétroxyde.

15. Procédé selon l'une quelconque des revendications précédentes caractérisé en ce que le composé de formule (I) donnée dans la revendication 1, utilisé est le cyclohexane.

16. Procédé selon l'une quelconque des revendications précédentes caractérisé en ce que l'hydroperoxyde utilisé est l'hydroperoxyde de cyclohexyle.

**17.** Procédé selon l'une quelconque des revendications précédentes caractérisé en ce que la réaction est conduite en phase liquide.

## Patentansprüche

**1.** Verfahren zur katalytischen Oxidation von Verbindungen der allgemeinen Formel (I):

RH (I)

in der R darstellt:
- eine lineare oder verzweigte Alkylgruppe mit 1 bis 30 Kohlenstoffatomen,
- eine Cycloalkylgruppe mit 3 bis 12 Kohlenstoffatomen im Ring, gegebenenfalls substituiert durch ein oder mehrere Alkylgruppen, die höchstens 4 Kohlenstoffatome umfassen,
- eine Polycycloalkylgruppe mit 2 bis 5 Ringen, wobei jeder Ring 3 bis 12 Kohlenstoffatome umfassen kann, oder
- eine alkyl- oder cycloalkylaromatische Gruppe, die 7 bis 30 Kohlenstoffatome umfaßt,

in einer Mischung von Alkoholen und Ketonen durch ein organisches Hydroperoxid in Anwesenheit eines Katalysators auf der Basis von Osmium, dadurch gekennzeichnet, daß das Osmium in Form von metallischem Osmium, gegebenenfalls auf einen Träger aufgebracht, in Form einer mineralischen Osmium-Verbindung oder in Form eines organischen Osmium-Komplexes eingetragen wird, der in dem Reaktionsmedium eine mineralische Form des Osmiums freisetzt, und dadurch, daß man bei einer Temperatur zwischen 50 und 180 °C arbeitet.

**2.** Verfahren nach Anspruch 1, dadurch gekennzeichnet, daß das organische Hydroperoxid die allgemeine Formel (II)

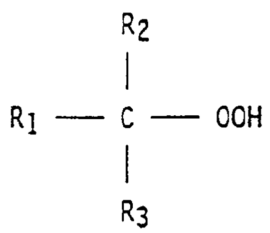

(II)

aufweist, in der:
- $R_1$ bis $R_3$, gleich oder verschieden, darstellen:
  - * Wasserstoffatome,
  - * lineare oder verzweigte Alkylgruppen, die 1 bis 30 Kohlenstoffatome umfassen,
  - * Cycloalkylgruppen, die 3 bis 12 Kohlenstoffatome umfassen,
  - * alkyl- oder cycloalkylaromatische Gruppen, die 7 bis 30 Kohlenstoffatome umfassen, oder
  - * Arylgruppen, gegebenenfalls substituiert durch ein oder zwei Alkylgruppen, die höchstens 4 Kohlenstoffatome umfassen, Arylgruppen, die 6 bis 20 Kohlenstoffatome umfassen,
  - * zwei der Gruppen $R_1$ bis $R_3$ können außerdem zusammen einen einzigen divalenten Alkylenrest bilden, der 4 bis 10 Kohlenstoffatome umfaßt.

**3.** Verfahren nach irgendeinem der vorstehenden Ansprüche, dadurch gekennzeichnet, daß die in Anspruch 1 angegebenen Verbindungen der Formel (I) ausgewählt werden unter denen, in denen R darstellt:
- eine lineare oder verzweigte Alkylgruppe mit 1 bis 12 Kohlenstoffatomen,
- eine Cycloalkylgruppe mit 5 bis 12 Kohlenstoffatomen im Ring,
- einen Alkylbenzolrest, in dem die Alkylgruppe höchstens 4 Kohlenstoffatome umfaßt,
- einen Cycloalkylbenzolrest, in dem die Cycloalkylgruppe 5 bis 8 Kohlenstoffatome umfaßt.

**4.** Verfahren nach irgendeinem der vorstehenden Ansprüche, dadurch gekennzeichnet, daß das in Anspruch 2 angegebene organische Hydroperoxid der Formel (II) entspricht, in der $R_1$ bis $R_3$, gleich oder verschieden, darstellen:
- Alkylgruppen mit 1 bis 4 Kohlenstoffatomen,
- Phenylgruppen, oder

- zwei von ihnen einen einzigen divalenten Rest, der mit dem Kohlenstoff, an den sie gebunden sind, einen Cyclohexylrest oder einen Cyclooctylrest bildet, oder
- eines von ihnen ein Wasserstoffatom.

**5.** Verfahren nach irgendeinem der vorstehenden Ansprüche, dadurch gekennzeichnet, daß man in Anwesenheit eines Verdünnungsmittels arbeitet, ausgewählt unter den gesättigten Alkoholen, den gesättigten Diolen und Polyolen, Wasser sowie ihren Mischungen.

**6.** Verfahren nach Anspruch 5, dadurch gekennzeichnet, daß man in Anwesenheit eines Alkohols der Formel (III)

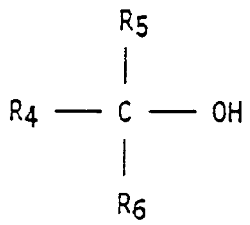

(III)

arbeitet, in der
- $R_4$ bis $R_6$, gleich oder verschieden, darstellen:
  * Wasserstoffatome,
  * lineare oder verzweigte Alkylgruppen mit 1 bis 30 Kohlenstoffatomen,
  * Cycloalkylgruppen mit 3 bis 12 Kohlenstoffatomen,
  * alkyl- oder cycloalkylaromatische Gruppen mit 7 bis 30 Kohlenstoffatomen,
  * Arylgruppen, gegebenenfalls substituiert durch ein oder zwei Alkylgruppen, die höchstens 4 Kohlenstoffatome umfassen, Arylgruppen, die 6 bis 20 Kohlenstoffatome umfassen;
  * zwei der Gruppen $R_4$ bis $R_6$ können außerdem zusammen einen einzigen divalenten Alkylenrest bilden, der 4 bis 10 Kohlenstoffatome umfaßt,
- alle drei Gruppen $R_4$ bis $R_6$ können außerdem zusammen einen einzigen trivalenten polycyclischen Rest bilden, der 6 bis 20 Kohlenstoffatome umfaßt.

**7.** Verfahren nach Anspruch 6, dadurch gekennzeichnet, daß der Alkohol der Formel (III) entspricht, in der $R_4$ bis $R_6$, gleich oder verschieden, lineare Alkylgruppen darstellen, die 1 bis 4 Kohlenstoffatome oder ein Wasserstoffatom umfassen.

**8.** Verfahren nach irgendeinem der Ansprüche 5 bis 7, dadurch gekennzeichnet, daß der Alkohol 2 bis 200 %, vorzugsweise 2 bis 100 Gewichtsprozent der in Anspruch 1 angegebenen Verbindung der Formel (I) repräsentiert.

**9.** Verfahren nach irgendeinem der Ansprüche 5 bis 8, dadurch gekennzeichnet, daß man in Anwesenheit einer Mischung von Alkohol und Wasser arbeitet.

**10.** Verfahren nach irgendeinem der Ansprüche 5 bis 9, dadurch gekennzeichnet, daß der verwendete Alkohol tert.-Butanol ist.

**11.** Verfahren nach irgendeinem der vorstehenden Ansprüche, dadurch gekennzeichnet, daß die Menge an Osmium zwischen $10^{-2}$ und $10^{-5}$ Mol pro Mol Hydroperoxid beträgt.

**12.** Verfahren nach irgendeinem der vorstehenden Ansprüche, dadurch gekennzeichnet, daß das organische Hydroperoxid 0,001 bis 100 Molprozent, vorzugsweise 0,01 bis 25 Molprozent, der in Anspruch 1 angegebenen Verbindung der Formel (I) beträgt.

**13.** Verfahren nach irgendeinem der vorstehenden Ansprüche, dadurch gekennzeichnet, daß die Reaktionstemperatur zwischen 70 °C und 150 °C beträgt.

**14.** Verfahren nach irgendeinem der vorstehenden Ansprüche, dadurch gekennzeichnet, daß das Osmium in Form seines Tetroxides eingetragen wird.

**15.** Verfahren nach irgendeinem der vorstehenden Ansprüche, dadurch gekennzeichnet, daß die in Anspruch 1 angegebene, verwendete Verbindung der Formel (I) Cyclohexan ist.

**16.** Verfahren nach irgendeinem der vorstehenden Ansprüche, dadurch gekennzeichnet, daß das verwendete Hydroperoxid Cyclohexyl-hydroperoxid ist.

**17.** Verfahren nach irgendeinem der vorstehenden Ansprüche, dadurch gekennzeichnet, daß die Reaktion in flüssiger Phase durchgeführt wird.

## Claims

**1.** Process for the catalytic oxidation of compounds of the general formula

RH (I)

in which R represents:

- a linear or branched alkyl group having 1 to 30 carbon atoms,
- a cycloalkyl group having 3 to 12 carbon atoms in the ring, optionally substituted by one or more alkyl groups containing up to 4 carbon atoms,
- a polycycloalkyl group comprising from 2 to 5 rings each of which may contain 3 to 12 carbon atoms, or
- an alkyl- or cycloalkylaromatic group having 7 to 30 carbon atoms,

to a mixture of alcohols and ketones with an organic hydroperoxide in the presence of an osmium-based catalyst, characterised in that the osmium is introduced in the form of metallic osmium, if necessary deposited on a support, in the form of an inorganic compound of osmium or in the form of an organic complex of osmium which releases an inorganic form of osmium into the reaction medium, and in that the process is carried out at a temperature of between 50 and 180°C.

**2.** Process according to Claim 1, characterised in that the organic hydroperoxide has the general formula:

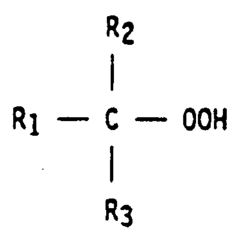

(II)

in which:

- $R_1$ to $R_3$, which may be the same or different, represent:
  - * hydrogen atoms,
  - * linear or branched alkyl groups having 1 to 30 carbon atoms,
  - * cycloalkyl groups having 3 to 12 carbon atoms,
  - * alkyl- or cycloalkylaromatic groups having 7 to 30 carbon atoms, or
  - * aryl groups, optionally substituted by one or two alkyl groups containing up to 4 carbon atoms, aryl groups having 6 to 20 carbon atoms;
- two of the $R_1$ to $R_3$ groups together may moreover form a single divalent alkylene radical containing 4 to 10 carbon atoms.

**3.** Process according to any one of the preceding claims, characterised in that the compounds of formula (I) given in Claim 1 are selected from among those for which R represents:

- a linear or branched alkyl group having 1 to 12 carbon atoms,
- a cycloalkyl group having 5 to 12 carbon atoms in the ring,
- an alkylbenzene residue in which the alkyl group contains up to 4 carbon atoms, or
- a cycloalkylbenzene residue in which the cycloalkyl group contains 5 to 8 carbon atoms.

**4.** Process according to any one of the preceding claims, characterised in that the organic hydroperoxide has the formula (II) given in Claim 2 in which $R_1$ to $R_3$, which may be the same or different, represent:

- alkyl groups having 1 to 4 carbon atoms
- phenyl groups, or
- for two of them a single divalent radical forming with the carbon atom to which it is attached a cyclohexyl or cyclooctyl residue, or
- for one of them, a hydrogen atom.

**5.** Process according to any one of the preceding claims, characterised in that the process is carried out in the presence of a diluent selected among the saturated alcohols, the saturated diols and polyols, water and mixtures of these.

**6.** Process according to Claim 5, characterised in that the process is carried out in the presence of an alcohol of formula

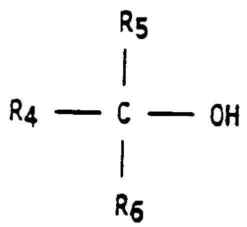

(III)

in which:

- $R_4$ to $R_6$, which may be indentical or different, represent:
  - ∗ hydrogen atoms,
  - ∗ linear or branched alkyl groups having 1 to 30 carbon atoms,
  - ∗ cycloalkyl groups having 3 to 12 carbon atoms,
  - ∗ alkyl- or cycloalkylaromatic groups having 7 to 30 carbon atoms, or
  - ∗ aryl groups, optionally substituted by one or two substituent alkyl groups containing up to 4 carbon atoms, aryl groups having 6 to 20 carbon atoms;
- two of the $R_4$ to $R_6$ groups together may moreover form a single divalent alkylene radical containing 4 to 10 carbon atoms,
- the three $R_4$ to $R_6$ groups together may moreover form a single trivalent polycyclic radical containing 6 to 20 carbon atoms.

**7.** Process according to Claim 6, characterised in that the alcohol has the formula (III) in which $R_4$ to $R_6$, which may be identical or different, represent linear alkyl groups having 1 to 4 carbon atoms or a hydrogen atom.

**8.** Process according to any one of Claims 5 to 7, characterised in that the alcohol represents 2 to 200 % and preferably 2 to 100 % relative to the weight of the compound of formula (I) given in Claim 1.

**9.** Process according to any one of Claims 5 to 8, characterised in that the process is carried out in the presence of a mixture of alcohol and water.

**10.** Process according to any one of claims 5 to 9, characterised in that the alcohol used is tert-butanol.

**11.** Process according to any one of the preceding claims, characterised in that the quantity of osmium is between $10^{-2}$ and $10^{-5}$ mole per mole of hydroperoxide.

**12.** Process according to any one of the preceding claims, characterised in that the organic hydroperoxide represents from 0.001 to 100 % (molar) of the compound of formula (I) given in Claim 1, and preferably from 0.01 to 25 % (molar).

**13.** Process according to any one of the preceding claims, characterised in that the reaction temperature is between 70 and 150°C.

**14.** Process according to any one of the preceding claims, characterised in that the osmium is introduced in the form of its tetroxide.

**15.** Process according to any one of the preceding claims, characterised in that the compound of formula (I) given in claim 1 which is used is cyclohexane.

**16.** Process according to any one of the preceding claims, characterised in that the hydroperoxide used is cyclohexyl hydroperoxide.

**17.** Process according to any one of the preceding claims, characterised in that the reaction is conducted in the liquid phase.